# EUROPEAN PATENT SPECIFICATION

(11) **EP 3 345 595 B1**
(45) Date of publication and mention of the grant of the patent: **08.04.2020**
(21) Application number: 16842020.6
(22) Date of filing: 02.09.2016
(51) Int. Cl.: A61K 31/198, A23K 20/142, A23L 33/175, A61P 21/06

(54) **COMPOSITION FOR MUSCLE BUILDING AND METHOD FOR BUILDING MUSCLE**
ZUSAMMENSETZUNG FÜR MUSKELAUFBAU UND VERFAHREN ZUM MUSKELAUFBAU
COMPOSITION POUR LE DÉVELOPPEMENT MUSCULAIRE ET MÉTHODE DE DÉVELOPPEMENT MUSCULAIRE

(30) Priority: 04.09.2015 JP 2015175052
(43) Date of publication of application: 11.07.2018
(73) Proprietor: University of Tsukuba, Tsukuba-shi, Ibaraki 305-8577 (JP); KYOWA HAKKO BIO CO., LTD., Tokyo 100-8185 (JP)
(72) Inventor: OKURA, Tomohiro, Tsukuba-shi Ibaraki 305-8577 (JP); YOON, Ji-Yeong, Tsukuba-shi Ibaraki 305-8577 (JP); SUZUKI, Takashi, Tokyo 100-8185 (JP); KOMATSU, Miho, Tokyo 100-8185 (JP); IKEDA, Takeshi, Tokyo 100-8185 (JP); KAMIMURA, Ayako, Tokyo 100-8185 (JP)
(74) Representative: Vossius & Partner Patentanwälte Rechtsanwälte mbB
(86) International application number: PCT/JP2016/075871
(87) International publication number: WO 2017/038991

(56) References cited:
- JP-A- 2010 506 887
- JP-A- 2011 509 293
- US-A1- 2007 248 648
- DATABASE GNPD [Online] MINTEL; 1 August 2012 (2012-08-01), anonymous: "Amino Acid Drink", XP055536675, retrieved from www.gnpd.com Database accession no. 1853376
- DATABASE GNPD [Online] MINTEL; 27 July 2011 (2011-07-27), anonymous: "Amino Acid Charge Tablets", XP055536684, retrieved from www.gnpd.com Database accession no. 1593963

## Description

### Technical Field

The present invention relates to a composition for building muscle, for example, a pharmaceutical product, a food (a concept including a food additive), and a feed (a concept including a feed additive) for building muscle, and further relates to a method for building muscle.

### Background Art

For example, a decrease in muscle mass is observed in those who tend to lack exercise, etc. also due to the effect of aging. In particular, it is extremely difficult for elderly people to perform sufficient exercise for enhancing muscle mass. Elderly people generally have low muscle mass, and in order to live safely, enhancing muscle mass or avoiding decrease in muscle mass is sometimes desired. Further, athletes necessary try to supply nutrition effectively in order to build muscle, and sometimes use a pharmaceutical product, a supplement, or the like.

The use of steroidal agents and growth hormone for muscle building is known, however, these have a problem with doping and side effects. In addition, compositions having a muscle building activity have been reported (Patent Documents 1 to 4).

### Citation List

### Patent Document

Patent Document 1: JP-A-2004-168704
Patent Document 2: JP-A-2004-292325
Patent Document 3: JP-A-2004-256513
Patent Document 4: WO 2004/075905

US 2007/248648 discloses a composition comprising 1-15 % of citrulline malate and 0,01-5 wt-% of branched-chain amino acids.

### Summary of Invention

### Problems to be Solved by the Invention

An object of the present invention is to provide a composition for building muscle. Further, another object of the present invention is to provide a method for building muscle of an animal whose muscle are intended to be built.

### Means for Solving the Problems

The present inventors made intensive studies in order to solve the above objects, and as a result, they found that a composition containing citrulline or a salt thereof and a branched-chain amino acid or a salt thereof has an excellent muscle building effect, and thus completed the present invention.

That is, the present invention is as follows.
(1) A composition for building muscle comprising citrulline or a salt thereof and a branched-chain amino acid or a salt thereof as active ingredients.
(2) The composition for building muscle described in (1), wherein the branched-chain amino acid is leucine.
(3) The composition for building muscle described in (1) or (2), wherein compositions in the form of agricultural and marine products or processed products thereof are excluded.
(4) The composition for building muscle described in (1) or (2), wherein the composition is a pharmaceutical product.
(5) The composition for building muscle described in any one of (1) to (3), wherein the composition is a food.
(6) The composition for building muscle described in any one of (1) to (5), wherein the composition is for an elderly people.
(7) The composition for building muscle described in any one of (1) to (3), wherein the composition is a feed.
(8) A method for building muscle comprising a step of allowing a subject animal whose muscle are intended to be built to ingest citrulline or a salt thereof and a branched-chain amino acid or a salt thereof in an amount necessary for building muscle of the subject animal.

In the composition and the method according to the invention, the amount of citrulline or a salt thereof in terms of free citrulline is from 10 wt% to 40 wt% of the composition and the total amount of branched-chain amino acid(s) or salt(s) thereof in terms of free branched amino acid(s) is from 20 wt% to 80 wt% of the composition.

### Effects of the Invention

According to the present invention, a composition for building muscle such as a pharmaceutical product, a food, or a feed effective in building muscle or ameliorate muscle weakening can be provided. Further, according to the present invention, a method for building muscle of an animal including a human can be provided.

### Brief Description of Drawings

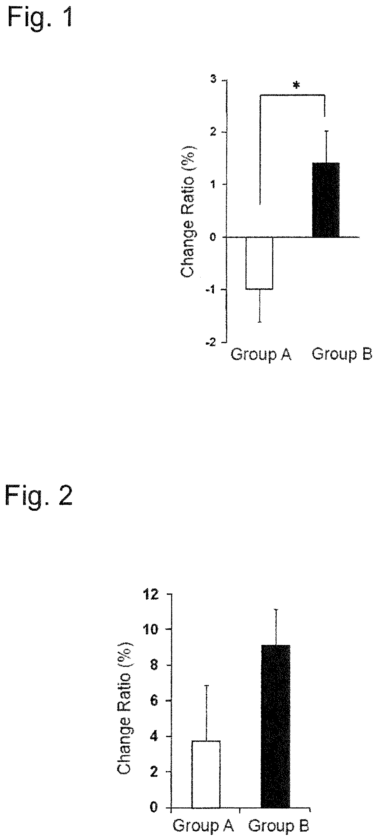

[FIG. 1] FIG. 1 shows the ratio of change (%) of whole body mass after 10 weeks vs start period of a test. In FIG. 1, in the horizontal axis, group A performed exercise (N=11), and group B performed exercise and also ingesting a food of Example 1 (N=6). The vertical axis represents the ratio of change (%) in the muscle mass of the whole body 10 weeks after the start of the test relative to before the start of the test, and a bar represents the mean ± standard deviation of the ratio of change.
[FIG. 2] FIG. 2 shows the ratio of change (%) of grip strength after 10 weeks vs start period of a test. In FIG. 2, in the horizontal axis, group A performed exercise (N=11), and group B performed exercise and also ingesting a food of Example 1 (N=6). The vertical axis represents the ratio of change (%) in grip strength 10 weeks after the start of the test relative to before the start of the test, and a bar represents the mean ± standard deviation of the ratio of change.

### Embodiments for Carrying Out the Invention

In the present invention, the "building muscle" refers to mainly increasing the skeletal muscle mass or the muscular strength and encompasses increasing the muscle weight, etc. Further, a decrease in muscle mass or muscular strength is referred to as muscle weakening, however, the building muscle as used herein is a concept also encompassing ameliorating muscle weakening.

The composition for building muscle of the present invention (hereinafter sometimes referred to as "composition of the present invention") contains citrulline or a salt thereof (hereinafter sometimes referred to as "component (a)") and a branched-chain amino acid or a salt thereof (hereinafter sometimes referred to as "component (b)") as active ingredients.

Examples of citrulline used in the composition of the present invention include L-citrulline, D-citrulline, and DL-citrulline, and L-citrulline is preferred. Citrulline can be obtained by a chemical synthesis method, a fermentation production method, or the like. Further, citrulline can also be obtained by purchasing a commercially available product. Examples of the chemical synthesis method for citrulline include the methods described in J. Biol. Chem., 122, 477 (1938) and J. Org. Chem., 6, 410 (1941).

Examples of the fermentation production method for L-citrulline include the methods described in JP-A-53-075387 and JP-A-63-068091. Further, L-citrulline, D-citrulline, and DL-citrulline can also be purchased from various companies.

Examples of the salt of citrulline include acid addition salts, metal salts, ammonium salts, organic ammonium salts, organic amine addition salts, amino acid addition salts, and the like.

Examples of the acid addition salts include inorganic acid salts such as hydrochlorides, sulfates, nitrates, and phosphates, and organic acid salts such as acetates, maleates, fumarates, citrates, malates, lactates, α-ketoglutarates, gluconates, and caprylates.

Examples of the metal salts include alkali metal salts such as sodium salts and potassium salts, alkaline earth metal salts such as magnesium salts and calcium salts, aluminum salts, zinc salts, and the like.

Examples of the organic ammonium salts include tetramethylammonium salts and the like. Examples of the organic amine addition salts include monoethanolamine salts, diethanolamine salts, triethanolamine salts, morpholine salts, piperidine salts, and the like. Examples of the amino acid addition salts include glycine salts, phenylalanine salts, lysine salts, aspartate salts, glutamate salts, and the like.

Among the above-mentioned salts of citrulline, a citrate or a malate is preferably used for the objects of the present invention, however, another salt may be used, and also, two or more salts may be appropriately combined and used. Incidentally, the above-mentioned salts of citrulline can be produced according to a method known per se and used, however, commercially available products provided from various companies can also be used.

The branched-chain amino acid used in the composition of the present invention is an amino acid having a branched aliphatic chain, and examples thereof include valine, leucine, and isoleucine. It is possible to use one type or two or more types in combination among these, and leucine is particularly preferred. As the amino acid, any of a D-form, an L-form, and a DL-form can be used, however, an L-form is preferred.

Further, as the branched-chain amino acid, one extracted and purified from an animal, a plant, or the like, one obtained by chemical synthesis, one produced by a fermentation method, or the like can be used, and commercially available products provided from various companies can also be used.

Examples of the salt of the above-mentioned branched-chain amino acid include the same salts as described with respect to citrulline, and it can be produced by a method known per se and used, and further, commercially available products provided from various companies can be used.

The composition of the present invention can be provided in a general formulation in the pharmaceutical technical field such as a liquid, an emulsion, a gel, a capsule, a powder, a granule, or a tablet.

The contents of the component (a) and the component (b) in the composition of the present invention vary depending also on the mode of administration or the like of the composition, however, as for the component (a), the amount in terms of free citrulline is generally from 10 wt% to 40 wt%, preferably from 15 wt% to 30 wt%. Further, as for the component (b), the total amount in terms of free branched amino acid is generally from 20 wt% to 80 wt%, preferably from 30 wt% to 60 wt%.

As for the content ratio between the component (a) and the component (b) in the composition of the present invention, the amount of the component (b) (the total amount in terms of free branched amino acid) with respect to 1 part by weight of the component (a) (the amount in terms of free citrulline) is generally from 1 part by weight to 3 parts by weight, preferably from 1.5 parts by weight to 2.5 parts by weight.

In the composition of the present invention, compositions in the form of agricultural and marine products or processed products thereof are excluded. The agricultural and marine products refer to agricultural products which are products by agriculture and marine products which become foods or food ingredients among aquatic living organisms such as fishes and shellfishes or seaweeds collected from the oceans, rivers, lakes and marshes, or the like, and the processed products thereof refer to foods or food ingredients produced by utilizing the agricultural and marine products. Therefore, in the present invention, the sentence "compositions in the form of agricultural and marine products or processed products thereof are excluded" means that compositions in the form of foods or food ingredients eaten or taken in daily common meal are excluded.

The composition for building muscle of the present invention can be provided as a pharmaceutical product for building muscle (hereinafter sometimes referred to as "pharmaceutical product of the present invention"). The pharmaceutical product of the present invention contains citrulline or a salt thereof and a branched-chain amino acid or a salt thereof as active ingredients, and may further contain any other active ingredient for treatment. The pharmaceutical product of the present invention is produced by any method well known in the pharmaceutical technical field by mixing the active ingredients together with a carrier.

As the mode of administration of the pharmaceutical product of the present invention, one which is most effective in the treatment is preferably used, and examples thereof include oral administration, and parenteral administration such as intravenous, intraperitoneal, and subcutaneous administration, however, oral administration is preferred because administration can be performed simply and easily.

Examples of the formulation to be administered include oral preparations such as a tablet, a powder, a granule, a pill, a suspension, an emulsion, an infusion/decoction, a capsule, a syrup, a liquid, an elixir, an extract, a tincture, and a fluidextract, and parenteral preparations such as an injection, drops, a cream, and a suppository, however, oral preparations are preferred.

When the composition is formulated as an oral preparation, a general additive such as an excipient, a binder, a disintegrant, a lubricant, a dispersant, a suspending agent, an emulsifier, a diluent, a buffer agent, an antioxidant, a preservative, or a flavoring agent can be used.

For example, a preparation preferred for oral administration such as a tablet, a powder, or a granule can be formulated by adding an excipient such as a saccharide such as lactose, white soft sugar, glucose, sucrose, mannitol, or sorbitol, starch such as potato, wheat, or corn starch, an inorganic material such as calcium carbonate, calcium sulfate, sodium hydrogen carbonate, or sodium chloride, crystalline cellulose, or a plant powder such as licorice powder or gentian powder, a disintegrant such as starch, agar, gelatin powder, crystalline cellulose, caramellose sodium, caramellose calcium, calcium carbonate, sodium hydrogen carbonate, or sodium alginate, a lubricant such as magnesium stearate, talc, hydrogenated vegetable oil, Macrogol, or silicone oil, a binder such as polyvinyl alcohol, hydroxypropyl cellulose, methyl cellulose, ethyl cellulose, caramellose, gelatin, or a starch paste, a surfactant such as a fatty acid ester, a plasticizer such as glycerin, or the like.

A preferred preparation for parenteral administration such as an injection can be formulated using a carrier composed of a salt solution, a glucose solution, or a mixture of a salt solution and a glucose solution, or the like. Further, also to these parenteral preparations, one or more additives selected from a diluent, a preservative, a flavoring agent, an excipient, a disintegrant, a lubricant, a binder, a surfactant, a plasticizer, etc. exemplified for the oral preparation can be added.

The contents of the component (a) and the component (b) in the pharmaceutical product of the present invention vary depending also on the formulation or the like of the pharmaceutical product, however, as for the component (a), the amount in terms of free citrulline is generally from 10 wt% to 40 wt%, preferably from 15 wt% to 30 wt%. Further, as for the component (b), the total amount in terms of free branched amino acid is generally from 20 wt% to 80 wt%, preferably from 30 wt% to 60 wt%.

The dose and the dosing frequency of the pharmaceutical product of the present invention depend on the mode of formulation, the age and body weight of a patient, the nature or severity of the symptoms to be treated, etc., however, in general, the pharmaceutical product is administered to an adult (body weight: 60 kg) once to several times a day in such an amount that the total amount of the component (a) and the component (b) (in terms of free citrulline and branched amino acid) is from 1 g to 10 g, preferably from 2 g to 6 g per day. The administration period is not particularly limited, but is typically 1 day to 1 year, preferably 2 weeks to 3 months.

Further, the composition of the present invention can be used not only for a human, but also an animal other than humans (hereinafter referred to as "nonhuman animal"). Examples of the nonhuman animal include animals other than humans such as mammals, birds, reptiles, amphibians, and fishes.

Therefore, the pharmaceutical product of the present invention can also be provided as a pharmaceutical product for building muscle of a nonhuman animal. The dose of the pharmaceutical product of the present invention when it is administered to a nonhuman animal varies depending on the species and age of the animal, the nature or severity of the symptoms, etc., however, in general, the pharmaceutical product is administered once to several times a day in such an amount that the total amount of the component (a) and the component (b) (in terms of free citrulline and branched amino acid) is from 0.02 g to 0.2 g, preferably from 0.03 g to 0.1 g per kilogram of body weight per day.

The administration period is not particularly limited, but is typically 1 day to 1 year, preferably 2 weeks to 3 months.

The composition of the present invention can also be provided as a food for building muscle (hereinafter sometimes referred to as "food of the present invention"). Incidentally, the food of the present invention also encompasses a food additive.

The food additive which is one form of the food of the present invention (hereinafter sometimes referred to as "food additive of the present invention") can be produced by the same method as that for the pharmaceutical preparation of the present invention. The food additive of the present invention can be processed and produced in the form of, for example, a powder, a granule, a pellet, a tablet, or various types of liquids.

The food of the present invention may be in any form of, for example, beverages such as juice, soft drinks, teas, and lactic acid bacteria drinks, dairy products such as fermented milk, butter, cheese, yogurt, processed milk, and skim milk, livestock meat products such as ham, sausages, and hamburger steaks, fish meat paste products such as kamaboko (boiled fish paste loaf), chikuwa (boiled fish paste tubular roll), and Satsuma-age (deep fried fish paste cake), egg products such as dashi-maki (Japanese rolled omelet) and egg tofu, confectionery such as frozen or chilled sweets, cookies, jellies, chewing gums, candies, and snack confectionery, bread, noodles, tsukemono (Japanese pickles), smoked products, dried foods, tsukudani (foods boiled in sweetened soy sauce), salted food products, soups, seasonings, etc., and may also be a bottle food, a canned food, or a retort-pouched food. Further, the food of the present invention may be in the same form as the pharmaceutical product of the present invention.

Further, the food of the present invention may be in the form of, for example, a powder, a sheet, a capsule, a tablet, a jelly, or the like. In addition, the food of the present invention can be provided as a food for specified health uses for building muscle, a health functional food such as a nutritional functional food or a food or beverage with function claims, a special-use food such as a food for a patient or a food for an elderly person, a health supplementary food, or the like.

To the food of the present invention and the food additive of the present invention, an additive which is generally used in foods, for example, a sweetener, a colorant, a preservative, a thickening stabilizer, an antioxidant, a color developing agent, a bleaching agent, an antifungal agent, a gum base, a bitter agent, an enzyme, a brightening agent, a sour agent, a seasoning, an emulsifier, a toughening agent, a production agent, a flavor, a spice extract, or the like described in Handbook for Indication of Food Additives (The Japan Food Additives Association, issued on Jan. 6, 1997) may be added.

The food of the present invention can be processed and produced by using a general method for producing a food except that the component (a) and the component (b) are added to a food or these components are added in the form of the above-mentioned food additive. Further, the food of the present invention can also be produced by a modified method of the method for producing the pharmaceutical product of the present invention.

Further, in a case where the food of the present invention is a solid food such as a granule, it can also be produced by using, for example, a granulation method such as fluid bed granulation, stirring granulation, extrusion granulation, oscillating granulation, gas stream granulation, compression molding granulation, disruption granulation, spray granulation, or jet granulation, a coating method such as pan coating, fluid bed coating, or dry coating, a puffing method such as puff drying, an excess steam method, a foam mat method, or a microwave heating method, an extrusion method such as using an extruding granulator or an extruder, or the like.

The addition amounts of the component (a) and the component (b) into the food of the present invention are appropriately determined according to the type of food, an effect expected by ingestion of the food, or the like, however, the component (a) and the component (b) are added in such amounts that the total amount of the component (a) and the component (b) (in terms of free citrulline and branched amino acid) is generally from 0.1 wt% to 20 wt%, preferably from 1 wt% to 10 wt%. Further, the contents of the component (a) and the component (b) in the food additive of the present invention are set such that when the food additive of the present invention is added to a food, the contents of the component (a) and the component (b) in the food fall within the above-mentioned range.

The ingestion amount, the ingestion frequency, and the ingestion period of the food of the present invention are the same as the dose and the like of the pharmaceutical product of the present invention for a human.

In a case where the food of the present invention is provided as a health functional food, a special-use food, a health supplementary food, or the like, it is preferred that the ingestion amount per meal calculated from the above-mentioned ingestion amount of the food of the present invention is contained in the food in a form in which the food is packed or filled in a unit of ingestion amount per meal. Here, the "food in a form in which the food is packed or filled in a unit of ingestion amount per meal" means that the food in an amount to be ingested per meal is packed or filled in one container such as bag, box, or bottle.

The composition of the present invention can also be provided as a feed for building muscle (hereinafter sometimes referred to as "feed of the present invention"). Incidentally, the feed of the present invention also encompasses a feed additive.

The feed additive which is one form of the feed of the present invention (hereinafter sometimes referred to as "feed additive of the present invention") can be produced in accordance with the method for producing the pharmaceutical product of the present invention in the same manner as the food additive of the present invention. The feed additive of the present invention is processed and produced in the form of, for example, a powder, a granule, a pellet, a tablet, or various types of liquids by mixing or dissolving another feed additive as needed.

The feed of the present invention can be processed and produced by adding the component (a) and the component (b) or a feed additive containing these components to a feed for a nonhuman animal and using a general method for producing a feed.

The feed for a nonhuman animal may be any as long as it is a feed for a nonhuman animal such as a mammal, a bird, a reptile, an amphibian, or a fish, and examples thereof include feeds for pets such as dogs, cats, or hamsters, feeds for livestock such as cattle or pigs, feeds for poultry such as chickens or turkeys, feeds for farmed fishes such as tunas, sea breams, or young yellowtails, and the like.

Examples of the feed for a nonhuman animal which can be used in the present invention include cereals, chaff and bran, vegetable press cake, animal-derived feeds, other feeds, purified products, and the like.

Examples of the cereals include milo, wheat, barley, oat, rye, brown rice, buckwheat, foxtail millet, millet, barnyard millet, corn, soybean, and the like. Examples of the chaff and bran include rice bran, defatted rice bran, wheat bran, wheat germs, barley bran, corn bran, corn germs, and the like.

Examples of the vegetable press cake include soybean press cake, linseed press cake, cottonseed press cake, peanut press cake, safflower press cake, coconut press cake, palm press cake, sesame press cake, sunflower press cake, rapeseed press cake, kapok press cake, mustard press cake, and the like.

Examples of the animal-derived feeds include fish meal (for example, northern ocean meal, imported meal, whole meal, coastal meal, etc.), fish soluble, meat meal, meat and bone meal, blood meal, degraded hair, bone meal, processed by-products for livestock, feather meal, silkworm pupa, skim milk, dry whey, and the like.

Examples of the other feeds include stems and leaves of plants (for example, alfalfa, hay cube, alfalfa leaf meal, false acacia powder, etc.), processed industrial by-products of corn (for example, corn gluten meal, corn gluten feed, corn steep liquor, etc.), processed starch products (for example, starch, etc.), sugar, fermentation industrial products (for example, yeast, beer cake, malt root, alcohol cake, soy source cake, etc.), agricultural by-products (for example, processed citrus fruit cake, soybean curd cake, coffee cake, cocoa cake, etc.), cassava, broad bean, guar meal, seaweed, krill, spirulina, chlorella, minerals, and the like.

Examples of the purified products include proteins (for example, casein, albumin, etc.), amino acids, carbohydrates (for example, sucrose, glucose, etc.), minerals, vitamins, and the like.

Further, the feed of the present invention can also be produced as a feed in the form of a solid such as a granule by using, for example, a granulation method such as fluid bed granulation, stirring granulation, extrusion granulation, oscillating granulation, gas stream granulation, compression molding granulation, disruption granulation, spray granulation, or jet granulation, a coating method such as pan coating, fluid bed coating, or dry coating, a puffing method such as puff drying, an excess steam method, a foam mat method, or a microwave heating method, an extrusion method such as using an extruding granulator or an extruder, or the like.

The addition amounts of the component (a) and the component (b) into the feed of the present invention are appropriately determined according to the type of feed, an effect expected by ingestion of the feed, or the like, however, the component (a) and the component (b) are added in such amounts that the total amount of the component (a) and the component (b) (in terms of free citrulline and branched amino acid) is generally from 0.1 wt% to 20 wt%, preferably from 1 wt% to 10 wt%.

Further, the contents of the component (a) and the component (b) in the feed additive of the present invention are set such that when the feed additive of the present invention is added to a feed, the contents of the component (a) and the component (b) in the feed fall within the above-mentioned range.

The ingestion amount, the ingestion frequency, and the ingestion period of the feed of the present invention are the same as the dose and the like of the pharmaceutical preparation for a nonhuman animal.

The compositions such as the pharmaceutical product, the food, and the feed of the present invention as described above exhibit an excellent muscle building effect. Therefore, the composition of the present invention is preferably used for an athlete whose muscle mass is desired to be increased and whose muscular strength is desired to be improved or an elderly person in whom a decrease in muscle mass, a decrease in muscular strength, or the like is observed with aging. Incidentally, in the present invention, the "elderly person" generally refers to a human who is 60 years or older.

Further, the composition of the present invention is also preferably used for a nonhuman animal such as livestock or poultry raised for meat or labor, or a farmed fish whose muscle mass is desired to be increased.

The present invention further provides a method for building muscle of a human and a nonhuman animal (hereinafter sometimes referred to as "method of the present invention"). The method of the present invention includes a step of allowing a subject animal whose muscle are intended to be built to ingest citrulline or a salt thereof and a branched-chain amino acid or a salt thereof in an amount necessary for building muscle of the subject animal.

Further, the step may include a step of performing exercise with a higher load than daily life. Examples of the exercise which is preferred for an elderly person in whom a decrease in muscle mass is observed include muscular strength training such as walking, jogging, or stepping exercise, swimming, and the like. The frequency of the exercise can be generally once to seven times a week, preferably once to three times a week.

The subject animal whose muscle are built includes humans and nonhuman animals. In a case of humans, the method of the present invention is preferably applied to athletes whose muscle are desired to be built, or elderly people in whom a decrease in muscle mass or muscular strength is observed. Further, in a case of nonhuman animals, the method of the present invention is preferably applied to livestock such as mammals raised for meat such as cattle, pigs, sheep, goats, or rabbits, or mammals raised for labor such as alpacas, horses, mules, camels, or donkeys, poultry raised for meat such as ducks, chickens, geese, turkeys, or quails, farmed fishes such as tunas, sea breams, or young yellowtails, or the like.

In the method of the present invention, a subject animal is allowed to ingest citrulline or a salt thereof and a branched-chain amino acid or a salt thereof in the form of the pharmaceutical product, the food, or the feed of the present invention described above. The ingestion amounts of citrulline or a salt thereof and a branched-chain amino acid or a salt thereof are determined according to the species, age, symptoms, conditions, etc. of the subject animal, however, these components can be ingested in the same amount as the above-mentioned dose of the pharmaceutical product of the present invention for a human and a nonhuman animal at the above-mentioned frequency for the above-mentioned period.

According to the method of the present invention, the muscle of a human or a nonhuman animal, preferably the skeletal muscle of the whole body such as head, neck, back, upper limbs, chest, abdomen, or lower limbs can be built.

### Examples

Examples of the present invention are described below.

### [Example 1] Food for Building muscle

A food for building muscle in the form of a powder was produced and packed in portions of 3 g. The contents of the active ingredients per package of the food of Example 1 are as follows: L-citrulline: 0.8 g, L-leucine: 1.6 g, L-valine: 0.3 g, and L-isoleucine: 0.3 g.

### [Test Example 1]

A test was performed by a randomized open-label study as follows. Seventeen females who are aged from 65 to 80, have a low body weight, that is, have a body mass index (BMI) of 17 or more and less than 20 were divided into two groups: group A (11 females) and group B (6 females), and both groups were allowed to perform stepping exercise and the exercise which is focused on muscular strength training mainly for the lower part of the body once a week for 9 weeks. Further, only group B was allowed to ingest two packages of the food for building muscle of Example 1 per day (one package after breakfast and after lunch) by dissolving the food in water for 10 weeks.

When the effectiveness of the food of Example 1 of the present invention was evaluated, an analysis on the 11 test subjects in group A and the 6 test subjects in group B was performed as follows. The muscle mass of the whole body and the grip strength of each test subject at the start of the test and 10 weeks after the start of the test were evaluated using a body composition analyzer (TANITA CORPORATION, a professional multi-frequency body composition analyzer, MC-980A) and a hand dynamometer (Takei Scientific Instruments Co., Ltd., Grip-D digital hand dynamometer, Smedley type, T.K.K. 5401). The ratio of change (%) was calculated from the values at the start of the test and 10 weeks after the start of the test, and the results of determining the average and the standard deviation for each group are shown in FIGS. 1 and 2. A larger value of the ratio of change indicates that the muscle mass of the whole body and the grip strength were increased.

A statistical significant difference test was performed on the ratio of change 10 weeks after the start of the test from the start of the test by an unpaired T-test between both groups. The symbol "*" in FIG. 1 indicates that there is a significant difference at a critical rate of 5% (p<0.05) between group B and group A.

As shown in FIGS. 1 and 2, by ingesting the food of Example 1 the present invention containing L-citrulline and a branched amino acid such as L-leucine, the muscle mass of the whole body 10 weeks after the start of the test was significantly increased. Further, a tendency in which also the grip strength was increased was confirmed.

From the above results, it was shown that by ingesting the food of the present invention containing L-citrulline and a branched amino acid such as L-leucine, the muscle mass of the whole body and the grip strength are increased.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2015-175052) filed on September 4, 2015 and the entire contents of which are incorporated herein by reference. Further, all references cited herein are incorporated herein by reference in their entirety.

### Industrial Applicability

As described in detail above, according to the present invention, a composition for building muscle effective in building muscle of a humans and a nonhuman animal can be provided. Further, according to the present invention, a method effective in building muscle of a human and a nonhuman animal can be provided.

## Claims

1. A composition for use in building muscle comprising citrulline or a salt thereof and a branched-chain amino acid or a salt thereof as active ingredients, wherein the amount of citrulline or a salt thereof in terms of free citrulline is from 10 wt% to 40 wt% of the composition and the total amount of branched-chain amino acid(s) or salt(s) thereof in terms of free branched amino acid(s) is from 20 wt% to 80 wt% of the composition.

2. The composition for use according to claim 1, wherein the branched-chain amino acid is leucine.

3. The composition for use according to claim 1 or 2, wherein compositions in the form of agricultural and marine products or processed products thereof are excluded.

4. The composition for use according to claim 1 or 2, wherein the composition is a pharmaceutical product.

5. The composition for use according to any one of claims 1 to 3, wherein the composition is a food.

6. The composition for use according to any one of claims 1 to 3, wherein the composition is a feed.

7. The composition for use according to any one of claims 1 to 5, wherein the composition is for use in an elderly person.

8. A non-therapeutic use of a composition comprising citrulline or a salt thereof and a branched-chain amino acid or a salt thereof for building muscle, wherein the amount of citrulline or a salt thereof in terms of free citrulline is from 10 wt% to 40 wt% of the composition and the total amount of branched-chain amino acid(s) or salt(s) thereof in terms of free branched amino acid(s) is from 20 wt% to 80 wt% of the composition.

## Patentansprüche

1. Eine Zusammensetzung zur Verwendung beim Muskelaufbau, umfassend Citrullin oder ein Salz davon und eine verzweigtkettige Aminosäure oder ein Salz davon als Wirkstoffe, wobei die Menge an Citrullin oder eines Salzes davon, bezogen auf freies Citrullin, 10 Gew.-% bis 40 Gew.-% der Zusammensetzung beträgt und die Gesamtmenge an verzweigtkettiger (verzweigtkettigen) Aminosäure(n) oder eines Salzes (Salzen) davon, bezogen auf freie verzweigte Aminosäure(n) 20 Gew.-% bis 80 Gew.-% der Zusammensetzung beträgt.

2. Die Zusammensetzung zur Verwendung gemäß Anspruch 1, wobei die verzweigtkettige Aminosäure Leucin ist.

3. Die Zusammensetzung zur Verwendung gemäß Anspruch 1 oder 2, wobei Zusammensetzungen in Form von landwirtschaftlichen Erzeugnissen oder Meereserzeugnissen, oder verarbeitete Erzeugnisse davon, ausgeschlossen sind.

4. Die Zusammensetzung zur Verwendung gemäß Anspruch 1 oder 2, wobei die Zusammensetzung ein Arzneimittel ist.

5. Die Zusammensetzung zur Verwendung gemäß einem der Ansprüche 1 bis 3, wobei die Zusammensetzung ein Lebensmittel ist.

6. Die Zusammensetzung zur Verwendung gemäß einem der Ansprüche 1 bis 3, wobei die Zusammensetzung ein Futtermittel ist.

7. Die Zusammensetzung zur Verwendung gemäß einem der Ansprüche 1 bis 5, wobei die Zusammensetzung zur Verwendung bei einer älteren Person vorgesehen ist.

8. Eine nicht-therapeutische Verwendung einer Zusammensetzung, umfassend Citrullin oder ein Salz davon und eine verzweigtkettige Aminosäure oder ein Salz davon zum Muskelaufbau, wobei die Menge an Citrullin oder eines Salzes davon, bezogen auf freies Citrullin, 10 Gew.-% bis 40 Gew.-% der Zusammensetzung beträgt und die Gesamtmenge an verzweigtkettiger (verzweigtkettigen) Aminosäure(n) oder eines Salzes (Salzen) davon, bezogen auf freie verzweigte Aminosäure(n) 20 Gew.-% bis 80 Gew.-% der Zusammensetzung beträgt.

## Revendications

1. Composition pour utilisation dans le développement musculaire comprenant de la citrulline ou un sel de celle-ci et un acide aminé à chaîne ramifiée ou un sel de celui-ci en tant que principes actifs, dans laquelle la quantité de citrulline ou d'un sel de celle-ci en termes de citrulline libre est de 10 % en poids à 40 % en poids de la composition et la quantité totale d'acide(s) aminé(s) à chaîne ramifiée ou de sel(s) de celui-ci (ceux-ci) en termes d'acide(s) aminé(s) ramifié(s) libre(s) est de 20 % en poids à 80 % en poids de la composition.

2. Composition pour utilisation selon la revendication 1, dans laquelle l'acide aminé à chaîne ramifiée est de la leucine.

3. Composition pour utilisation selon la revendication 1 ou 2, dans laquelle les compositions sous la forme de produits agricoles et marins ou de produits transformés de ceux-ci sont exclues.

4. Composition pour utilisation selon la revendication 1 ou 2, dans laquelle la composition est un produit pharmaceutique.

5. Composition pour utilisation selon l'une quelconque des revendications 1 à 3, dans laquelle la composition est un aliment.

6. Composition pour utilisation selon l'une quelconque des revendications 1 à 3, dans laquelle la composition est un aliment pour animaux.

7. Composition pour utilisation selon l'une quelconque des revendications 1 à 5, dans laquelle la composition est pour utilisation chez une personne âgée.

8. Utilisation non thérapeutique d'une composition comprenant de la citrulline ou un sel de celle-ci et un acide aminé à chaîne ramifiée ou un sel de celui-ci pour le développement musculaire, dans laquelle la quantité de citrulline ou d'un sel de celle-ci en termes de citrulline libre est de 10 % en poids à 40 % en poids de la composition et la quantité totale d'acide(s) aminé(s) à chaîne ramifiée ou de sel(s) de celui-ci (ceux-ci) en termes d'acide(s) aminé(s) ramifié(s) libre(s) est de 20 % en poids à 80 % en poids de la composition.
